# EUROPEAN PATENT SPECIFICATION

(11) **EP 1 549 241 B1**
(45) Date of publication and mention of the grant of the patent: **26.05.2010**
(21) Application number: 03749693.2
(22) Date of filing: 17.09.2003
(51) Int. Cl.: A61B 19/00, A61M 25/02

(54) **LOW PROFILE INSTRUMENT IMMOBILIZER**
FLACHER INSTRUMENTENHALTER
DISPOSITIF D'IMMOBILISATION D'INSTRUMENT A BAS PROFIL

(30) Priority: 17.09.2002 US 411309 P
(43) Date of publication of application: 06.07.2005
(73) Proprietor: MEDTRONIC, INC., Minneapolis, MN 55432 (US)
(72) Inventor: SKAKOON, James, G., St. Paul, MN 55105 (US); SOLAR, Matthew, S., Indialantic, FL 32903 (US); MILLER, Thomas, I., Palm Bay, FL 32909 (US); HELMER, Patrick R., Sebastian, Florida 32958 (US); FREAS, Mark S., Palm Bay, Florida 32909 (US)
(74) Representative: Maiwald Patentanwalts GmbH
(86) International application number: PCT/US2003/028966
(87) International publication number: WO 2004/026161

(56) References cited:
- WO-A-00/18306
- WO-A-01/24709
- US-A- 4 579 120
- US-A- 4 699 616
- US-A- 5 221 264
- US-A- 5 267 970
- US-A- 5 423 848
- US-A- 5 464 446
- US-A1- 2001 027 271
- US-A1- 2002 052 610

## Description

### FIELD OF THE INVENTION

This document relates generally to instrument immobilizers, and more specifically, but not by way of limitation, to a low profile instrument immobilizer.

### BACKGROUND

Neurosurgery sometimes involves inserting an electrode (for recording brain signals or providing stimulating pulses) or other instrument (for example, a catheter for fluid aspiration or drug infusion) through a burr hole or other entry portal into a subject's brain toward a target region of the brain. In certain applications, there is a need to secure the electrode or other instrument in place after it has been introduced, potentially for an extended period of time. Moreover, in certain applications, other equipment (such as a trajectory guide and any associated equipment) is mounted to the patient's skull about the burr hole.

For these and other reasons, which will become apparent upon reading the following detailed description and viewing the drawings that form a part thereof, the present inventors have recognized an umnet need for low profile instrument immobilizer devices, tools, and methods that reduce or avoid patient discomfort and also better retain instruments.

The general state of the art includes the following references:

US Pat. 5,423,848, discloses a trocar having an obturator including a piercing tip, and a cannula for forming a passage in tissue that is punched by the piercing tip. The cannula is formed from a flexible tube or a coil tube and transforms and libitum in accordance with the shape of the surgical tool that is to be inserted into the body cavity through the cannula.

US Pat. 5,221,264, discloses a reduction port for a trocar sleeve including a plate having an aperture of a predetermined diameter, a pair of doors slidably mounted to the plate for reducing the size of the aperture, and a securing component fastened to the plate for removably attaching the plate to a proximal end of a laparoscopic trocar sleeve. Each plate is provided with a semicircular aperture having a diameter equal to the reduced diameter of the aperture.

Published US Patent Application 2001/027271 A1 discloses a method and apparatus for positioning a surgical instrument during stereotactic surgery using a guidance fixture. The guidance fixture includes an upper portion, which includes an instrument guide for moving the surgical instrument along a constrained trajectory relative to the upper portion, and includes an adjustable base supporting the upper portion, which includes a mounting base and an adjustment mechanism. The adjustable base can include a rotating collar attached to the mounting base and a pivoting collar coupling the rotating collar to the upper portion. A remote sensing device, such as a camera array sensing LEDs on the fixture, is used to determine the orientation of the upper portion of the guidance assembly in relation to the body.

Published US Patent Application 2002/052610 A1 forms the closest prior art and discloses devices and methods for accurate targeting, placement, and/or stabilization of an electrode or other instrument(s) into the brain or other body organ, such as to treat severe tremor or other neurological disorders. Targeting is performed using any form of image-guidance, including real-time MRI, CT, or frameless surgical navigation systems. This document discloses a base hoop sized and shaped to be disposed about a burr hole, the base hoop including an upper surface and a lower surface, the base hoop defining an access passage extending between the upper surface and the lower surface of the base, the base hoop including at least one slot opening on a side of the base hoop and at least one hole sized and shaped to receive a fastener therethrough to secure the base hoop.
This document further discloses first and second retaining members or cover pieces sliding toward and away from each other and able to engage each other and operable to retain at least one instrument therebetween.

### BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example, but not by way of limitation, various embodiments discussed in the present document.

Figure **1** is a top view illustrating generally, by way of example, but not by way of limitation, portions of a first instrument immobilizer.

Figure **2** is a top view illustrating generally an example of portions of the first instrument immobilizer after cover pieces have been partially inserted into respective slots in opposing sides of a hoop.

Figure **3** is a perspective view illustrating generally an example of portions of the first instrument immobilizer after the cover pieces have been partially inserted into the respective slots of the hoop.

Figure **4** is a side view illustrating generally an example of portions of the first instrument immobilizer after the cover pieces have been almost fully inserted into the respective slots of the hoop.

Figure **5** is a top view illustrating generally an example of portions of the first instrument immobilizer after the cover pieces have been fully inserted into the respective slots of the hoop.

Figure **6** is a perspective view illustrating generally, by way of example, but not by way of limitation, portions of a second instrument immobilizer.

Figure **7** is a side view of the second instrument immobilizer with a latch piece in an open position.

Figure **8** is a perspective view of the second instrument immobilizer with the latch piece in a closed position.

Figure **9** is a side view of the second instrument immobilizer with the latch piece in a closed position.

Figure **10** is a top view of a third instrument immobilizer.

Figure **11** is a top view of the third instrument immobilizer with a clasp in a closed position.

Figure **12** is a perspective view of the third instrument immobilizer with the clasp in its closed position.

Figure **13** is a side view of the third instrument immobilizer with the clasp in its open position.

Figure **14** is a side view of the third instrument immobilizer with the clasp in its closed position.

Figure **15** is a perspective view of a fourth instrument immobilizer with base pieces in an open position.

Figure **16** is a top view of the fourth instrument immobilizer with base pieces in a closed position, grasping and immobilizing a laterally bent electrode.

Figure **17** is a top view of a fifth instrument immobilizer.

Figure **18** is a top view illustrating the fifth instrument immobilizer where the first retaining member and second retaining member immobilize an instrument.

Figure **19** is a top view of a sixth instrument immobilizer.

Figure **20** is a top view illustrating the sixth instrument immobilizer where the socket slot and socket immobilize an instrument.

Figure **21** is a perspective view of another example of the lid for the sixth instrument immobilizer having multiple slots and corresponding sockets.

Figure **22** is a top view illustrating generally the first instrument immobilizer where the cover pieces immobilize an instrument.

Figure **23** is a perspective view of the second instrument immobilizer with the latch piece in the closed position where the latch piece immobilizes an instrument.

Figure **24** is a perspective view of the third instrument immobilizer with the clasp in the closed position where the clasp immobilizes an instrument.

Figure **25** is a block diagram illustrating generally, by way of example, and not by way of limitation, a first method to immobilize an instrument.

Figure **26** is a block diagram illustrating generally, by way of example, and not by way of limitation, a second method to immobilize an instrument.

Figure **27** is a block diagram illustrating generally, by way of example, and not by way of limitation, a third method to immobilize an instrument.

Figure **28** is a block diagram illustrating generally, by way of example, and not by way of limitation, a fourth method to immobilize an instrument.

Figure **29** is a block diagram illustrating generally, by way of example, and not by way of limitation, a fifth method to immobilize an instrument.

Figure **30** is a block diagram illustrating generally, by way of example, and not by way of limitation, a sixth method to immobilize an instrument.

### DETAILED DESCRIPTION

In the following detailed description, reference is made to the accompanying drawings that form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the scope of the present invention. The following detailed description is, therefore, not to be taken in a limiting sense, and the scope of the present invention is defined by the appended claims and their equivalents.

In this document, the terms "a" or "an" are used, as is common in patent documents, to include one or more than one.

### Example 1

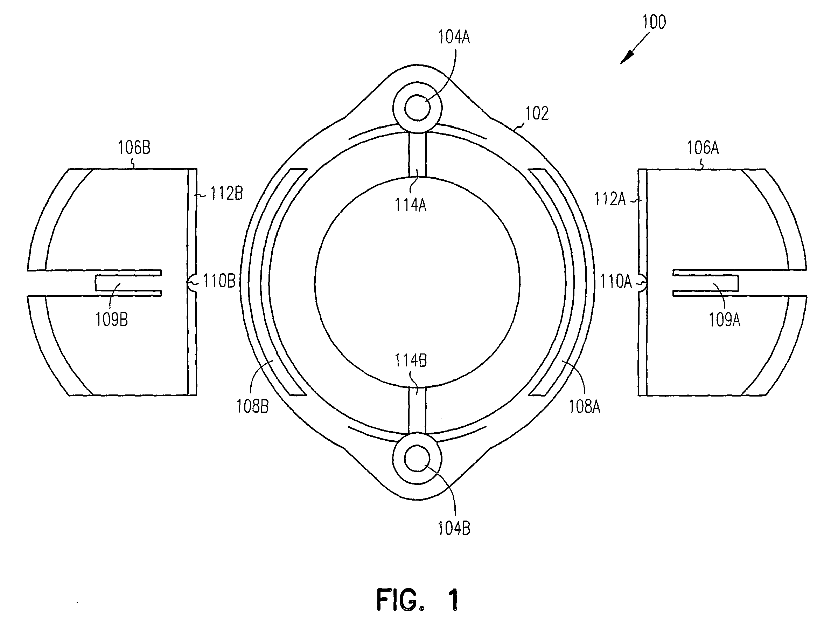

Figures **1 - 5** and **22** illustrate various views of a first embodiment of a low-profile instrument immobilizer for securing a flexible recording or stimulating electrode or the like, or other instrument (such as a catheter for fluid aspiration or drug, cell, or substance infusion) after it has been introduced through a burr hole or other entry portal to a desired target location in the brain.

Figure **1** is a top view illustrating generally, by way of example, but not by way of limitation, portions of instrument immobilizer **100.** In this example, instrument immobilizer **100** includes a hoop-like base **102** (also referred to herein as a "hoop"). The hoop **102** is sized and shaped for being circumferentially disposed about a burr hole (which, in this document, is understood to include a twist drill hole) or other entry portal of a desired size. Burr holes typically range in diameter between about 6 millimeters to about 14 millimeters. In this example, hoop **102** includes holes **104A-B** or other passages for receiving corresponding bone screws or the like therethrough for securing hoop **102** to a subject's skull (or other desired location upon the subject). In this example, hole **104A** is located on an opposite side of hoop **102** from hole **104B.** In operation, hoop **102** is secured to the skull before the electrode or other instrument is inserted to the desired location in the patient's brain. The electrode or other instrument (for example an aspiration catheter or an infusion catheter) is inserted through the circular center passage defined by hoop **102.**

In this example, instrument immobilizer **100** includes a two-piece sliding cover including cover pieces **106A-B.** Each cover piece **106A-B** is sized and shaped to be inserted through a corresponding side slot **108A-B** in an opposing side of hoop **102.** Each piece of cover piece **106A-B** also has a snap-fit or other catch **109A-B,** such as a deformable tab, that engages a portion of hoop **102** when that cover piece **106A** or **106B** has been fully inserted. Catches **109A-B** hold their corresponding cover piece **106A-B** in place after it has been fully inserted. Alternatively, the instrument immobilizer includes a one-piece sliding cover that, in one example, the single cover piece substantially covers the circular center passage when fully inserted through a corresponding side slot.

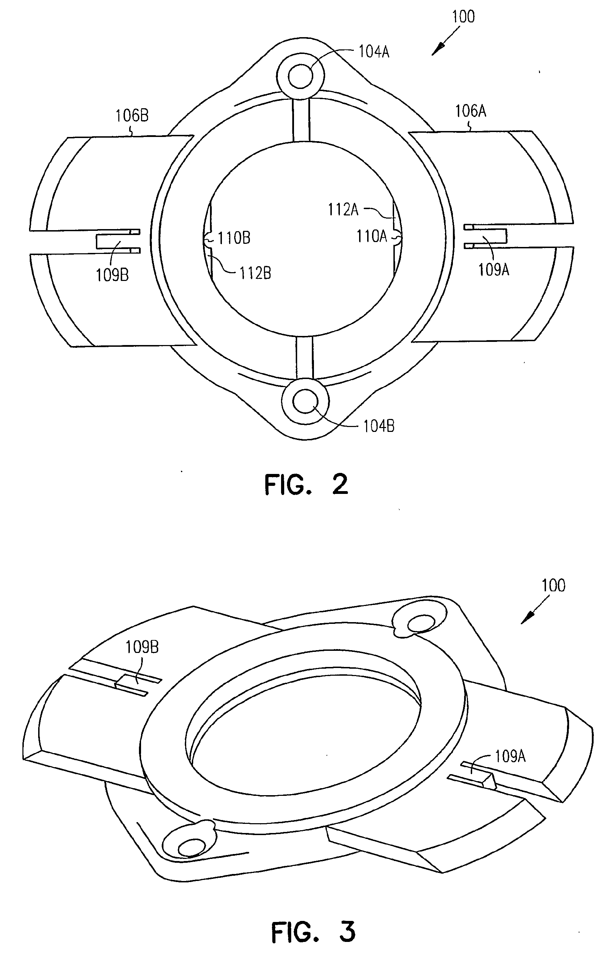

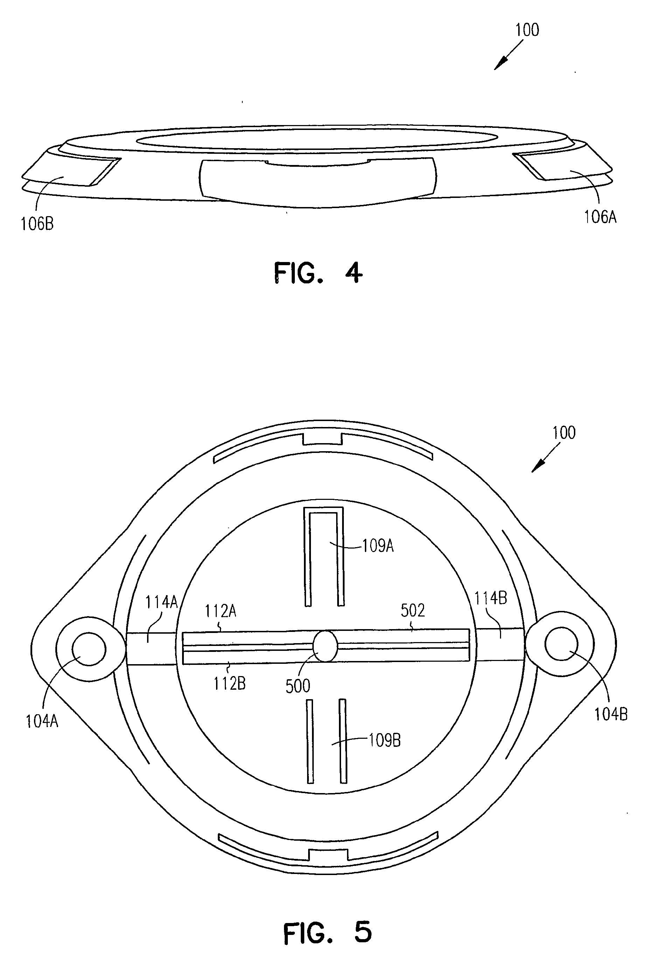

Figure **2** is a top view illustrating generally an example of portions of instrument immobilizer **100** after cover pieces **106A-B** have been partially inserted into respective slots **108A-B** in opposing sides of hoop **102.** Figure **3** is a perspective view illustrating generally an example of portions of instrument immobilizer **100** after cover pieces **106A-B** have been partially inserted into respective slots **108A-B** of hoop **102.** Figure **4** is a side view illustrating generally an example of portions of instrument immobilizer **100** after cover pieces **106A-B** have been almost fully inserted into respective slots **108A-B** of hoop **102.** Figure **5** is a top view illustrating generally an example of portions of instrument immobilizer **100** after cover pieces **106A-B** have been fully inserted into respective slots **108A-B** of hoop **102.**

As illustrated generally in Figures **1 - 5****,** when cover pieces **106A-B** have been fully inserted and pressed against each other, they substantially cover the center passage defined by hoop **102,** except for a small circular opening **500** formed by aligned semi-circular cutouts **110A-B** of cover pieces **106A-B.** Also, when cover pieces **106A-B** have been fully inserted and their respective beveled edges **112A-B** are pressed against each other, a resulting trough, channel or groove **502** is formed. In this example, groove **502** aligns with exit grooves **114A-B** in hoop **102,** however, this is not required. In another example, one or more exit grooves **114A-B** are disposed elsewhere about hoop **102,** such as, for example, exit grooves **114** distributed about hoop **102** at regular intervals (e.g., every 30 degrees, etc.). Moreover, such differently disposed exit grooves **114** are also applicable to all of the other examples depicted and/or described in this document.

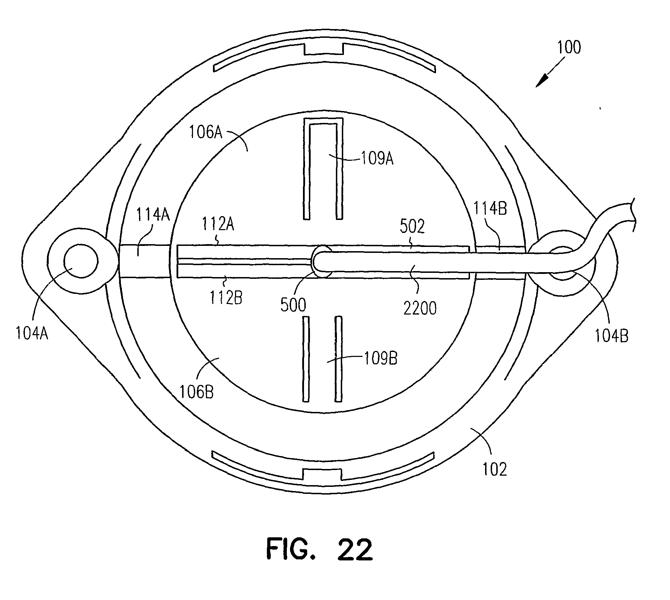

Figure **22** is a top view illustrating an instrument immobilizer **100** retaining at least one wire electrode or other introduced instrument **2200** (e.g., a catheter). Once the instrument **2200** is retained by cover pieces **106A-B** so as to immobilize the instrument, a portion of the instrument **2200** may be bent into a portion of groove **502** and one of exit grooves **114A-B** to laterally exit hoop **102.** In the above described example having multiple exit grooves **114A-B,** the instrument **2200** may be bent into any one of the exit grooves **114A-B.** The introduced instrument **2200** is retained and immobilized by the snug fit through opening **500.** Additionally the instrument **2200** is further retained by snug positioning of a portion of the instrument **2200** within the groove **502** and one of the exit grooves **114A-B.** Where the instrument **2200** is positioned within an exit groove **114A-B** the instrument immobilizer **100** and instrument **2200,** in one example, are covered by a cap that provides a semi-permanent fixture for both.

### Example 2

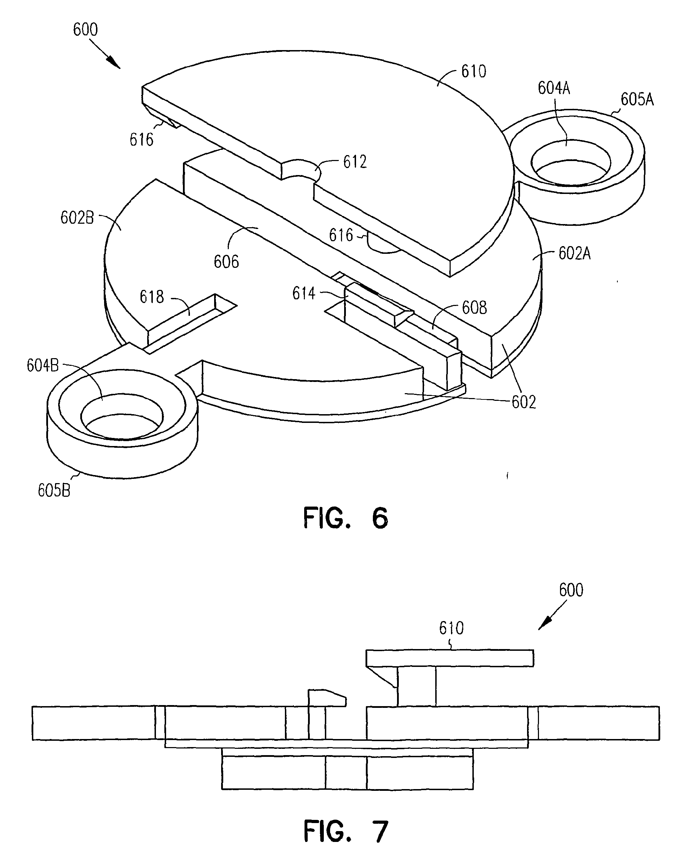

Figures **6 - 9** and **23** illustrate various views of a second embodiment of a low-profile instrument immobilizer for securing a flexible recording or stimulating electrode or the like (or other instrument, such as a catheter) after it has been introduced through a burr hole or other entry portal to a desired target location in the brain.

Figure **6** is a perspective view illustrating generally, by way of example, but not by way of limitation, portions of instrument immobilizer **600.** In this example, instrument immobilizer **600** includes a base **602** sized and shaped for being secured above and covering a similarly-sized burr hole or other entry portal. In this example base **602** includes a pair of semicircular pieces **602A-B.** Base **602** includes holes **604A-B** or other passages for receiving corresponding bone screws or the like therethrough for securing base **602** to a subject's skull (or other desired location upon the subject). In this example, hole **604A** is located on an opposite side of base **602** from hole **604B.** The holes **604A-B** are, in this example, located on members **605A-B,** which extend radially outward from base **602.** In one example of operation, base **602** is secured to the skull by inserting a screw through one of the holes **604A-B.** This permits the instrument immobilizer **600** to rotate about the axis of the screw through the one of the holes **604A-B.** The instrument immobilizer **600** is rotated away from the burr hole in this manner, thereby providing access to the burr hole. The electrode or other instrument is inserted through the burr hole to the target location, as desired. The instrument immobilizer is then rotated back toward the burr hole about the axis of the screw through the one of the holes **604A-B** such that the instrument extends through an access slot **606.** The instrument can then be further secured, such as discussed below.

In this example, the access slot **606** extends across base **602,** except for the presence of connecting member **608.** The slot **606** permits access to the underlying burr hole or other entry portal, except for the obstruction of connecting member **608**. Connecting member **608** provides a mechanical connection between two semicircular portions **602A-B** of base **602.** The semicircular portions **610A-B** are separated by slot **606** and connecting member **608.**

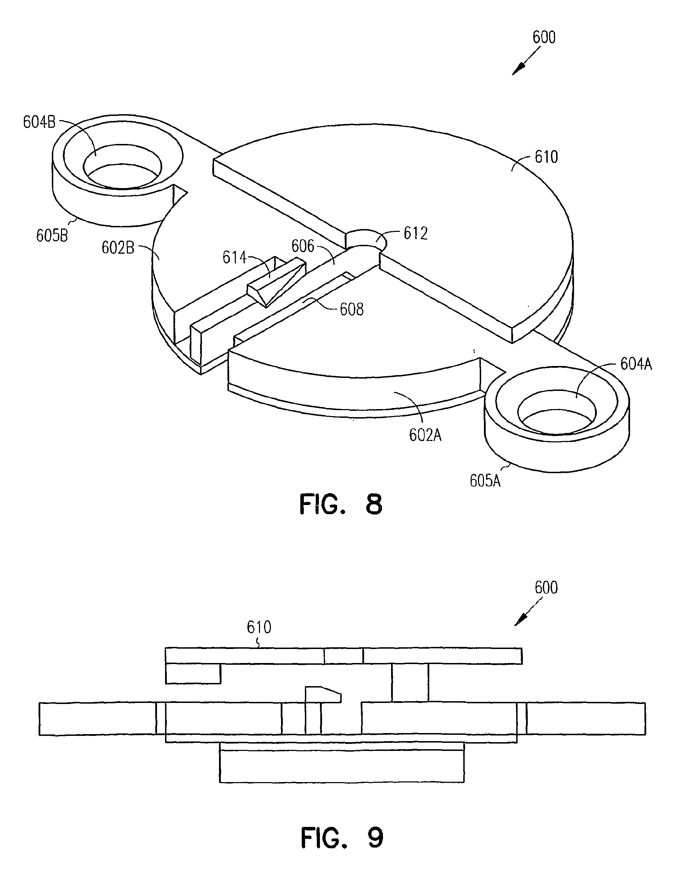

In this example, a latch **610** is eccentrically coupled to base **602** by coupling the latch to the base at a point offset from the center of the base. In the example of Figure **6****,** the latch includes a semicircular latch piece **610** having a post **616** that is snap-fit into semicircular piece **602A** of base **602.** Figure **6** illustrates latch piece **610** in an eccentrically-mounted open position. In this example, latch piece **610** includes a semicircular notch or cutout **612** that is sized to retain the electrode or other instrument snugly within slot **606** when latch piece **610** is moved to the closed position. After the instrument is axially positioned as desired within the slot **606,** then the latch piece **610** is moved to the closed position and immobilizes the instrument. Figure **7** is a side view of instrument immobilizer **600** with latch piece **610** in the open position. Figure **8** is a perspective view of instrument immobilizer **600** with latch piece **610** in the closed position. Figure **9** is a side view of instrument immobilizer **600** with latch piece **610** in the closed position.

In Figures **6 - 9****,** when latch piece **610** is in the closed position the semicircular shape of latch piece **610** aligns and conforms with the circumference of base **602** such that the peripheral profile of the latch piece **610** is coextensive with the peripheral profile of the base **602** when the immobilizer **600** is viewed from above.

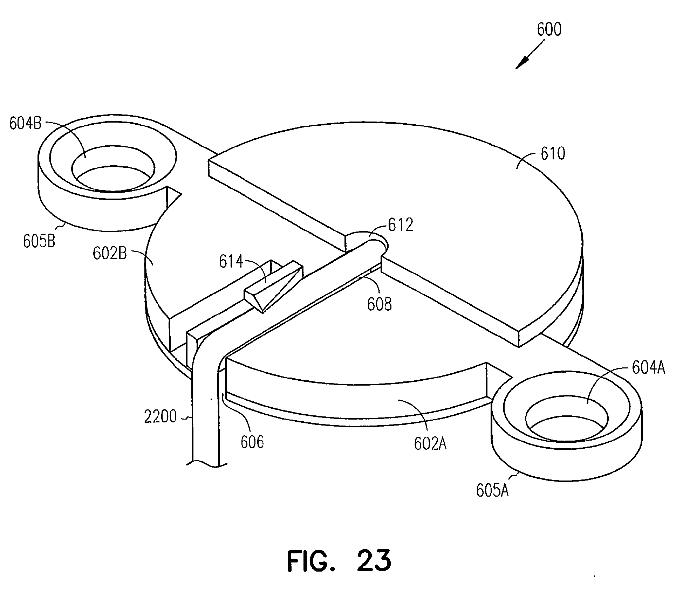

Figure **23** is a perspective view illustrating the instrument immobilizer retaining a wire electrode or other introduced instrument **2200** (for example a catheter as described above). In the closed position, the instrument **2200** is snugly retained within the semicircular cutout **612** between latch piece **610** and connecting member **608.** In this example, the latch piece **610** is securable in the closed position by a detent **616** that extends from a lower surface of the latch piece **610.** As generally shown in Figure **6****,** the detent **616** engages the base **602** within a securing slot **618** defined by the base to secure the latch piece **610** in the closed position. The instrument **2200** may be laterally bent (e.g., such as at about a 90 degree angle, or otherwise) to laterally exit base **602.** In this example, the laterally bent portion of the instrument **2200** may be wedged snugly under or along a tab (such as retaining member **614)** disposed above slot **606.** This further secures the instrument **2200** after immobilization by the latch piece **610.**

### Example 3

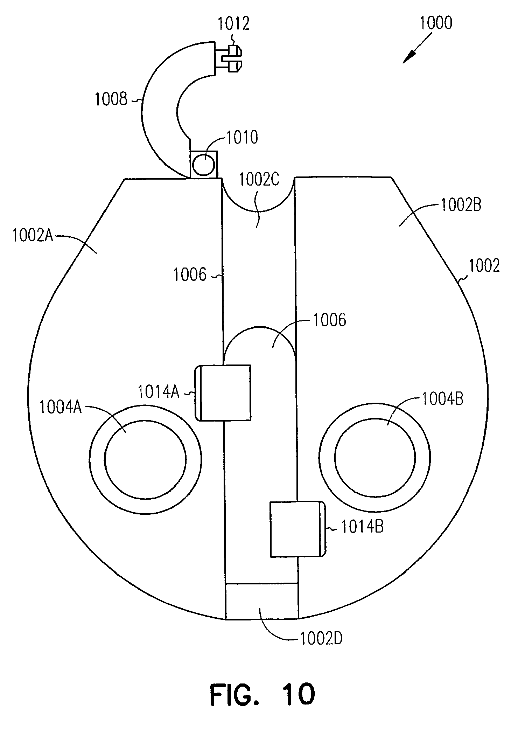

Figures **10 - 14** illustrate various views of a third embodiment of a low-profile instrument immobilizer for securing a flexible recording or stimulating electrode or the like, or other instrument, after it has been introduced through a burr hole or other entry portal to a desired target location in the brain.

Figure **10** is a top view illustrating generally, by way of example, but not by way of limitation, portions of instrument immobilizer **1000.** In this example, instrument immobilizer **1000** includes a base **1002** sized and shaped for being secured such that it is cantilevered over at least a portion of a burr hole or other entry portal. In this example base **1002** includes a pair of approximately semicircular portions **1002A-B** connected by connecting member portions **1002C-D.** Base **1002** includes holes **1004A-B** or other passages for receiving corresponding bone screws or the like therethrough for securing base **1002** to a subject's skull (or other desired location upon the subject). An optional slot or lateral groove **1006** extends between semicircular portions **1002A-B** and connecting member portions **1002C-D** of base **1002.**

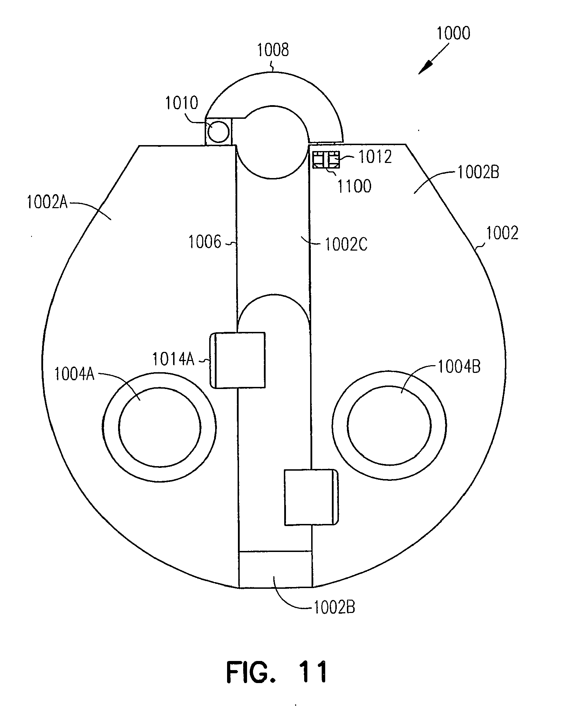

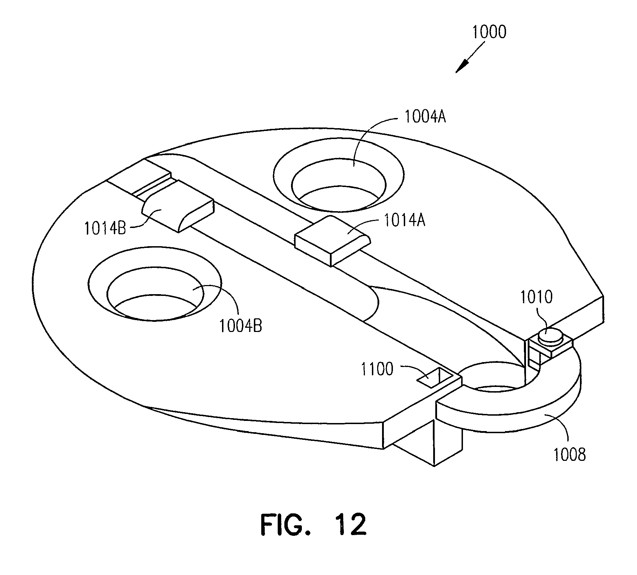

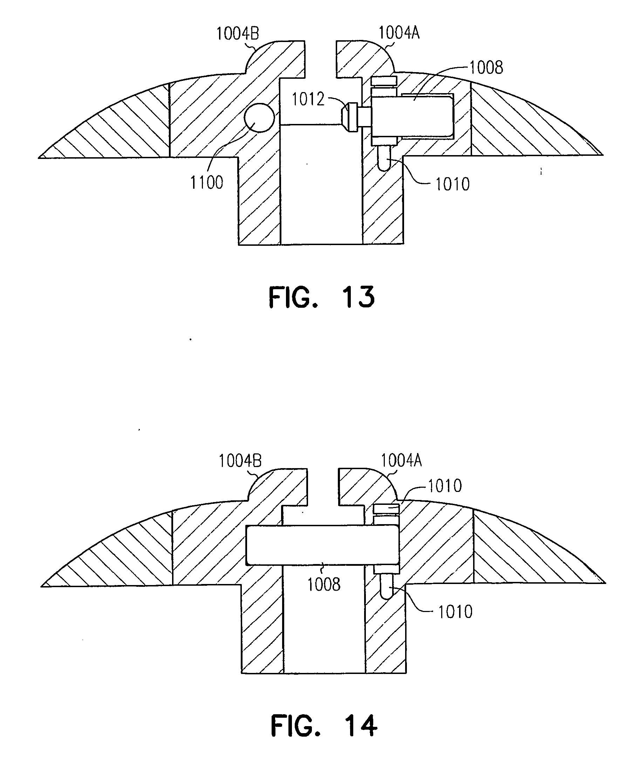

In this example, instrument immobilizer **1000** includes a semicircular shaped clasp **1008.** The clasp **1008** is coupled at a first end to one of the semicircular portions **1002A-B** by a hinge **1010.** At a second end, the clasp **1008** includes a fastener **1012.** In this example, the fastener **1012** includes a male snap-fitting, for securing to a mating female receptacle **1100** (Figure **11**)in the other of the semicircular portions **1002A-B** when the clasp **1008** is moved from an open position to a closed position. In another example, the clasp 1008 is coupled at a first end to one of the semicircular portions **1002A-B** with a flexible element, including but not limited to deformable plastic, or a hinge as described above. Figure **11** is a top view of instrument immobilizer **1000** with clasp **1008** in the closed position. Figure **12** is a perspective view of instrument immobilizer **1000** with clasp **1008** in its closed position. Figure **13** is a side view of instrument immobilizer **1000** with clasp **1008** in its open position. Figure **14** is a side view of instrument immobilizer **1000** with clasp **1008** in its closed position.

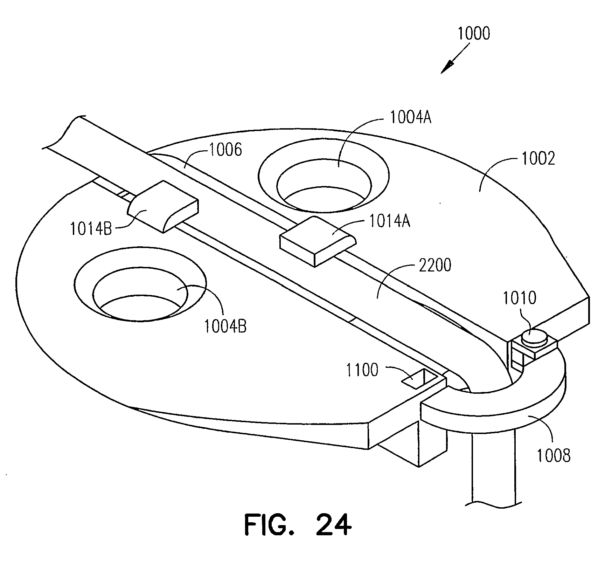

Figure **24** is a perspective view of an instrument immobilizer retaining a wire electrode or other instrument **2200** (for example, a catheter for aspiration or substance infusion). The instrument immobilizer base **1002** is secured to the skull such that clasp **1008** is cantilevered over the burr hole or other entry portal. The electrode or other instrument is inserted through the burr hole or other entry portal to the desired location so the instrument is near the clasp **1008.** In one option, the instrument is adjacent a side surface of the instrument immobilizer **1000.** The clasp **1008** is then closed around the electrode or other instrument to snugly retain it. The electrode or other instrument is laterally bent into lateral groove **1006** and positioned snugly under cantilevered retaining members **1014A-B** extending from semicircular portions **1002A-B** over lateral groove **1006.** As shown in Figure **24****,** The electrode or other instrument is thereby immobilized by clasp **1008.** Cantilevered retaining members **1014A-B** and/or lateral groove **1006** are sized or otherwise configured to snugly conform to the electrode or other instrument. This provides additional snug retention of the electrode or other instrument.

### Example 4

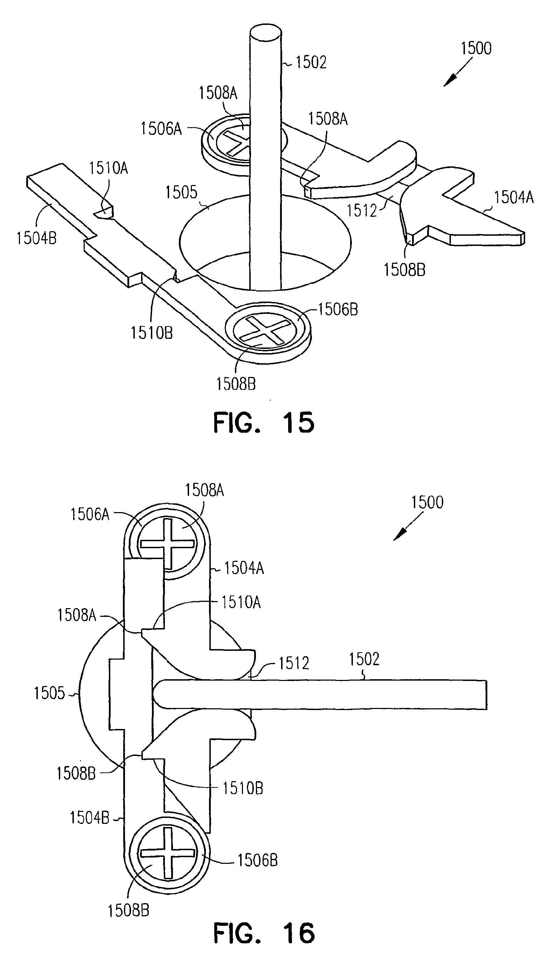

Figures **15 - 16** illustrate various views of a fourth embodiment of a low-profile instrument immobilizer for securing a flexible recording or stimulating electrode or the like, or other instrument (a catheter, in one example), after it has been introduced through a burr hole or other entry portal to a desired target location in the brain.

Figure **15** is a perspective view illustrating generally, by way of example, but not by way of limitation, portions of instrument immobilizer **1500** for securing an electrode **1502** or other instrument. In this example, instrument immobilizer **1500** includes a two-piece base **1504A-B.** Base piece **1504A** is sized and shaped for being disposed along a first side of a burr hole **1505** or other entry portal. Base piece **1504B** is sized and shaped for being disposed along a second side of a burr hole or other entry portal. Each elongated base piece **1504A-B** includes a corresponding hole **1506A-B,** at a first end, for receiving a corresponding bone screw **1508A-B** or the like therethrough for securing the corresponding base piece **1504A-B** to the subject's skull. In one example, the bone screws **1508A-B** and holes **1506A-B** are disposed on opposite sides of the burr hole **1505.** In another example, the base pieces **1504A-B** are sized and shaped so bone screws **1508A-B** and holes **1506A-B** are disposed at non-opposed locations around the burr hole **1505.** Each bone screw **1508A-B** and corresponding hole **1506A-B** provides a hinge about which the corresponding elongate base piece **1504A-B** rotates, such as from the open position illustrated in Figure **15** to a closed position, in which base pieces **1504A-B** retain and immobilize the electrode or other instrument. In one option, the base pieces **1504A-B** rotate about parallel axes. In another option the base pieces **1504A-B** rotate within the same plane. Figure **16** is a top view illustrating base pieces **1504A-B** in a closed position, retaining and immobilizing a laterally bent electrode **1502.**

In the example of Figures **15-16****,** base pieces **1504A-B** are disposed such that when these pieces are rotated into the closed position of Figure **16****,** male couplers **1508A-B** (for example snap fittings) on base piece **1504A** engage corresponding mating couplers **1510A-B** on base piece **1504B.** This fastens base pieces **1504A-B** together to retain electrode **1502** therebetween. Electrode **1502** is further laterally bent into the groove **1512** of base piece **1504A.** Groove **1512** is sized and shaped to snugly grasp electrode **1502** therein to further retain and immobilize electrode **1502.**

### Example 5

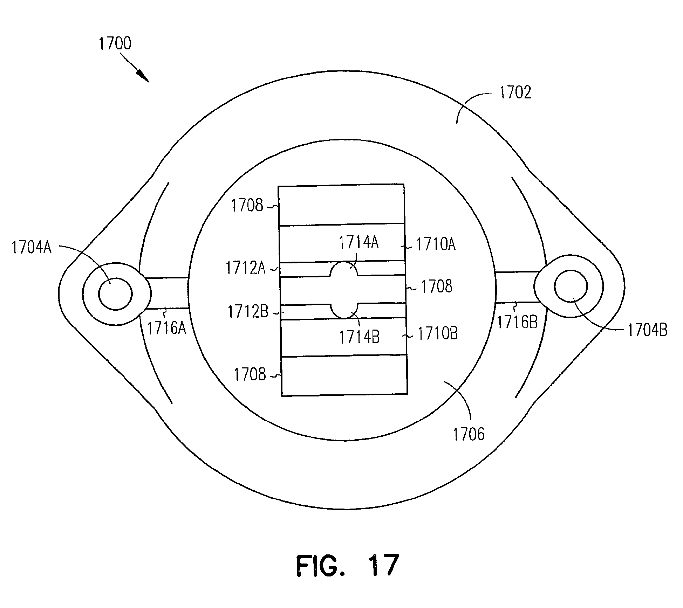

Figure **17** is a top view illustrating generally, by way of example, but not by way of limitation, a fifth embodiment of an instrument immobilizer, such as instrument immobilizer **1700.** In this example, instrument immobilizer **1700** includes a hoop-like base **1702** (also referred to herein as a "hoop") sized and shaped for being circumferentially disposed about a burr hole or other entry portal of a desired size. In this example, hoop **1702** includes holes **1704A-B** or other passages for receiving corresponding bone screws or the like therethrough for securing hoop **1702** to a subject's skull (or other desired location upon the subject). In this example, hole **1704A** is located on an opposite side of hoop **1702** from hole **1704B.**

In this example, hoop **1702** defines a center passage therewithin, into which an insert **1706** is disposed. Insert **1706** includes at least one snap-fitting feature that allows insert **1706** to be snapped into hoop **1702.** In one example, insert **1706** is inserted into the circular center passage of hoop **1702** such that insert **1706** rotates therewithin with respect to hoop **1702.** In this example, the insert **1706** includes at least one detent disposed about the insert bottom surface and projecting to the side of the insert. The hoop **1702** includes a corresponding grooved surface disposed upon an intermediate surface therein. The at least one detent engages the grooved surface. This engagement prevents unwanted rotation of the insert **1706** with respect to the hoop **1702.**

In Figure **17****,** insert **1706** includes a rectangular or other access opening **1708.** Retaining members **1710A-B** are movably coupled to the insert **1706.** In one example, the retaining members **1710A-B,** include slidable retaining members carried substantially within access opening **1708.** In one example, access opening **1708** includes opposing side slide rails that allow retaining members **1710A-B** to slide toward and away from each other within access opening **1708.** In the example of Figure **17****,** sliding retaining members **1710A-B** each have beveled edges **1712A-B** and semicircular cutouts **1714A-B** in center portions of beveled edges **1712A-B.** When retaining members **1710A-B** are positioned adjacent to each other, beveled edges **1712A-B** form a lateral groove, and semicircular cutouts **1714A-B** are aligned to each other to form a circular opening. In an alternative example, however, the semicircular cutouts **1714A-B** are omitted.

In one example, retaining members **1710A-B** include respective mating couplings to hold the retaining members in engagement with each other. In one option, the mating couplings include snap-fit features to maintain engagement between the retaining members **1710A-B.** In another example, at least one of the retaining members **1710A-B** includes an engagement coupling that secures the at least one of the retaining members **1710A-B** relative to insert **1706.** In one option, the engagement coupling includes a "one-way" toothed surface disposed substantially upon the side slide rails of the insert **1706.** Corresponding tabs are disposed along the bottom edge of the at least one of the retaining members **1710A-B.** The tabs engage the toothed surface and thereby allow slidable movement of the at least one of the retaining members **1710A-B** in one direction, typically toward the other retaining member. In another option, both retaining members **1710A-B** have tabs that engage toothed surfaces on the rails. When retaining members **1710A-B** contact each other, the tabs engaging the toothed rails maintain engagement between the retaining members **1710A-B.** The retaining members **1710A-B** do not disengage from each other because the tabs and teeth prevent sliding away in the other direction. If it is desirable to move the retaining members **1710A-B** in a direction counter to that allowed by the tabs and teeth the retaining members **1710A-B** are pulled out of engagement with the side rails, disengaged from each other and repositioned within the side slide rails.

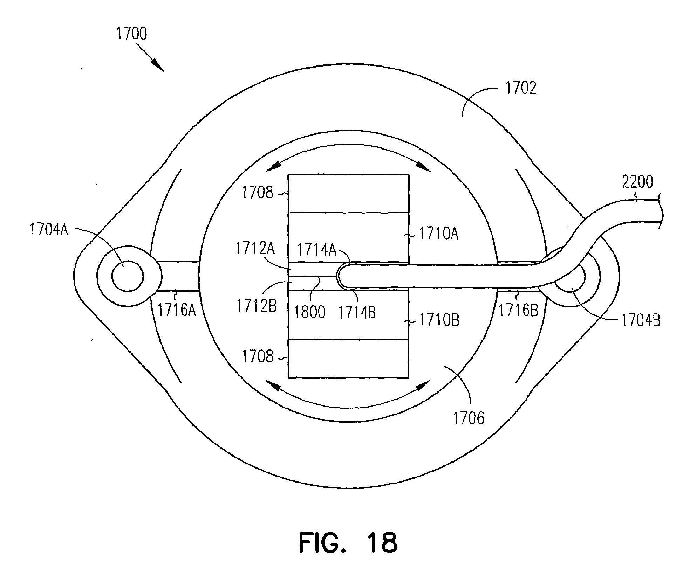

Figure **18** is a top view illustrating instrument immobilizer **1700** retaining a wire electrode or other instrument **2200.** In operation, hoop **1702** is secured and insert **1706** is inserted therein. An electrode or other instrument **2200** (for example an aspiration or infusion catheter) is inserted between retaining members **1710A-B** to the desired location in the patient's brain. In one example, the insert **1706** is capable of being rotated about the instrument **2200.** This provides added flexibility in the location for immobilization of the instrument. Retaining members **1710A-B** are moved against each other to snugly retain and immobilize a portion of the instrument **2200,** such as within the circular opening defined by semicircular cutouts **1714A-B.** In another example, the semicircular cutouts **1714A-B** are omitted and the instrument **2200** is compressibly retained between the beveled edges **1712A-B** at a desired position anywhere along the beveled edges. The retaining members **1710A-B** engage each other and/or the side rails (as discussed above) to immobilize the instrument **2200.** If desired, the instrument **2200** can be bent into the lateral groove formed **1800** by beveled edges **1712A-B.** In one example, the lateral groove **1800** aligns with one or more lateral exit grooves **1716A-B** providing lateral access through hoop **1702,** allowing the electrode or other instrument to laterally exit hoop **1702.** In one example, the lateral groove **1800** and/or the lateral exit groove(s) **1716A-B** are sized and shaped to snugly grasp and retain the instrument **2200.** In another example, once the instrument **2200** is immobilized by the retaining member **1710A-B** and grasped and retained by the lateral groove **1800** and/or lateral exit groove **1716A-B,** a cap is affixed to the instrument immobilizer **1700.**

### Example 6

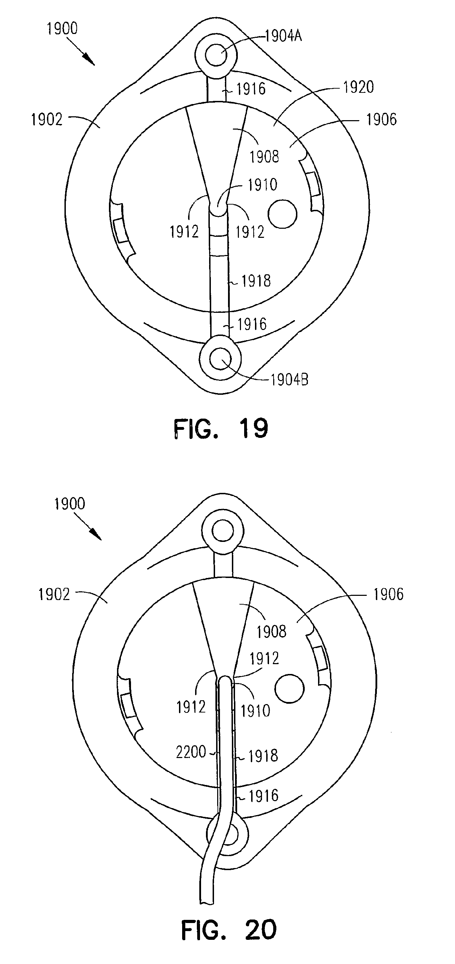

Figure **19** is a top view illustrating generally, by way of example, but not by way of limitation a sixth embodiment of an instrument immobilizer, such as instrument immobilizer **1900.** In this example, instrument immobilizer **1900** includes a hoop-like base **1902** (also referred to herein as a "hoop") sized and shaped for being circumferentially disposed about a burr hole or other entry portal of a desired size. In this example, hoop **1902** includes holes **1904A-B** or other passages for receiving corresponding bone screws or the like therethrough for securing hoop **1902** to a subject's skull (or other desired location upon the subject). In this example, hole **1904A** is located on an opposite side of hoop **1902** from hole **1904B.**

In this example, hoop **1902** defines a center passage therewithin, into which a lid **1906** is disposed. Lid **1906** includes at least one snap-fitting feature that allows lid **1906** to be snapped into hoop **1902.** In one example, lid **1906** rotates substantially within the circular center passage of hoop **1902.** In another example, the lid **1906** includes at least one detent on its bottom surface, and the hoop **1902** includes a corresponding groove on or near its inner periphery. The at least one detent engages the groove. This prevents undesirable rotation of the lid **1906** with respect to the hoop **1902.**

In this example, the lid **1906** includes a slot **1908.** In one option, the slot **1908** has an arcuate geometry defined by the lid **1906** and extends across an angle of approximately 15 degrees. The slot **1908** extends from an outer edge **1920** of the lid **1906,** tapering inward toward a center **1922** of the lid **1906.** The slot includes at least one socket **1910.** Figure **19** illustrates the socket **1910** extending through the center of the lid **1906.** However, the socket **1910** may alternatively be offset from the center of the lid **1906** to provide added flexibility in positioning and immobilizing an instrument. In one example, the slot **1908** narrows as it extends from the outer edge **1920** of the lid **1906** toward the socket **1910.** In Figure **19****,** just before reaching the socket **1910,** the slot **1908** is slightly more narrow than the socket **1910** is wide. This provides a lip **1912** on the slot **1908** adjacent the socket **1910.**

Figure **20** is a top view illustrating a wire electrode or other instrument **2200** (e.g., a catheter, such as for aspiration or infusion of drug(s), cell(s) or other substances) that is immobilized by the instrument immobilizer **1900.** In one example of operation, hoop **1902** is first secured. The instrument **2200** is then inserted through the hoop **1902** to the desired location in the patient's brain. In one example, the lid **1906** is then inserted into the hoop **1902** such that the instrument **2200** is disposed within a slot **1908.** Inserting the lid **1906** after positioning the instrument **2200** provides additional flexibility for positioning the slot around the instrument **2200** so as to reduce or minimize any unwanted movement of the instrument **2200.** The instrument **2200** is then advanced laterally within the slot **1908** toward the socket **1910.** Upon reaching the lip **1912,** a portion of the instrument **2200** and/or the lip **1912** defonns slightly to permit the instrument **2200** to enter the socket **1910** for retention and immobilization by the socket **1910.** In one example, the hoop **1902** includes at least one lateral exit groove **1916** and the lid **1906** includes at least one lateral exit groove **1918.** The lid **1906** is positionable to permit groove **1918** to align with one of the lateral exit grooves **1916.** A portion of the instrument **2200** is then bent and positioned within the groove **1918** and lateral exit groove **1916** where it is retained snugly therein. This further immobilizes the instrument **2200.** In one example, a cap is affixed to the instrument immobilizer **1900.**

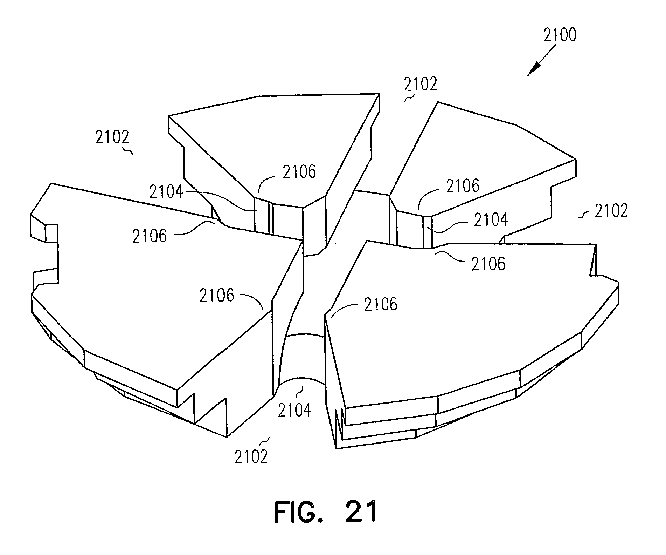

Figure **21** is a perspective view of another embodiment of a lid **2100** usable with the instrument immobilizer **1900** illustrating generally multiple slots **2102,** sockets **2104** and lips **2106.** The lid **2100,** as illustrated in the example of Figure **21****,** is operable to retain and immobilize up to three instruments. Alternately, the lid **2100** is operable to retain an instrument in multiple positions for flexibility of immobilization. Similar configurations can be used to immobilize fewer or greater instruments.

### Some Variations and Other Aspects of the Above Examples

In the above examples (aspects of which can be combined with each other), the components of instrument immobilizers 100,600, 1000,1500, 1700, 1900, and lid 2100 may be manufactured from molded plastic and are MRI compatible. The bone screws used for securing the instrument immobilizers may be manufactured from stainless steel. In one example, such bone screws include imaging fiducial markers integral or attachable thereto. Additionally, in other embodiments of the above examples the exit grooves are disposed at various locations (for example, at regular intervals) about the instrument immobilizers 100,600, 1000,1500, 1700, and 1900 to provide flexibility in the placement of the instruments when immobilized. Instrument immobilizers 100, 600,1000, 1500,1700, 1900, and lid 2100 are not limited to use in conjunction with skull burr holes in neurosurgery, but may be secured at other locations of a patient for securing an electrode or other surgical instrument (for example catheters used for aspiration or infusion), or about an entry portal in other objects into which an instrument has been introduced, and which requires immobilization. The above examples provide instrument immobilizers that are designed to advantageously provide a low-profile (e. g. , small height) above the patient's skull. By way of example, but not by way of limitation, in one embodiment, instrument immobilizer 100 provides a height of less than about 2.54 mm (0.1 inches), instrument immobilizer 600 provides a height of less than about 2.032 mm (0.08 inches), instrument immobilizer 1000 provides a height of less than about 2.032 mm (0.08 inches), instrument immobilizer 1500 provides a height of less than about 1.651 mm (0.065 inches), instrument immobilizer 1700 provides a height of less than about 2.54 mm (0.1 inches), and instrument immobilizer 1900 and lid 2100 provide a height of less than about 2.54 mm (0.1 inches).

### Method of Use Example 1

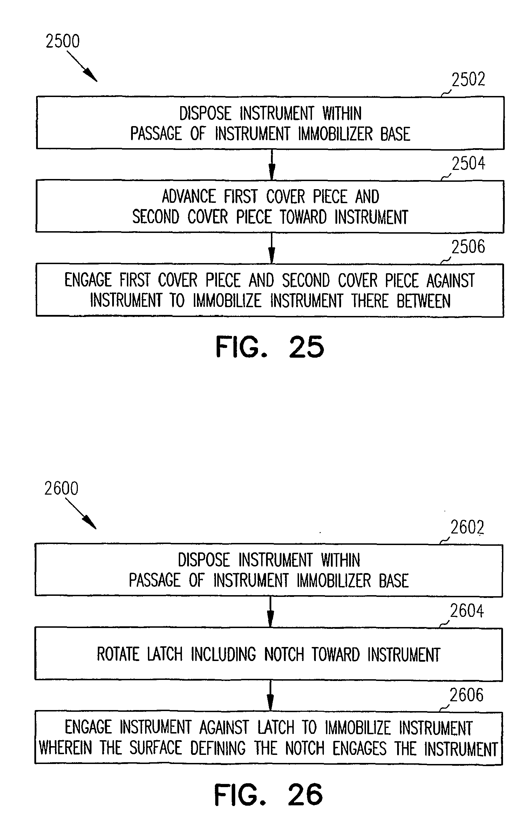

Figure 25 is a block diagram of a method 2500 for immobilizing an instrument, for example an electrode or catheter. At 2502, an instrument is disposed within a passage of an instrument immobilizer base. At 2504, a first cover piece and a second cover piece are advanced toward the instrument. At **2506** the first cover piece and the second cover piece are engaged against the instrument to immobilize the instrument therebetween.

Several variations are possible. One example includes sliding the first cover piece through a slot in the instrument immobilizer base and sliding the second cover piece through another slot in the instrument immobilizer base includes grasping and/or retaining a portion of the instrument within a groove defined by the first and second cover piece.

### Method of Use Example 2

Figure **26** is a block diagram of a method **2600** for immobilizing an instrument. At **2602,** an instrument is disposed within a passage of an instrument immobilizer base. At **2604,** a latch including a notch is rotated toward the instrument. At **2600** the instrument is engaged against the latch to immobilize the instrument, where the surface defining the notch engages the instrument.

Several variations are possible. One example includes retaining the latch against the instrument by engaging a portion of the latch to a mating portion of the instrument immobilizer base. In another example, a portion of the instrument is laterally positioned against the instrument immobilizer base, and the portion of the instrument is tucked under an overlying retaining member that holds the portion of the instrument against the instrument immobilizer base.

### Method of Use Example 3

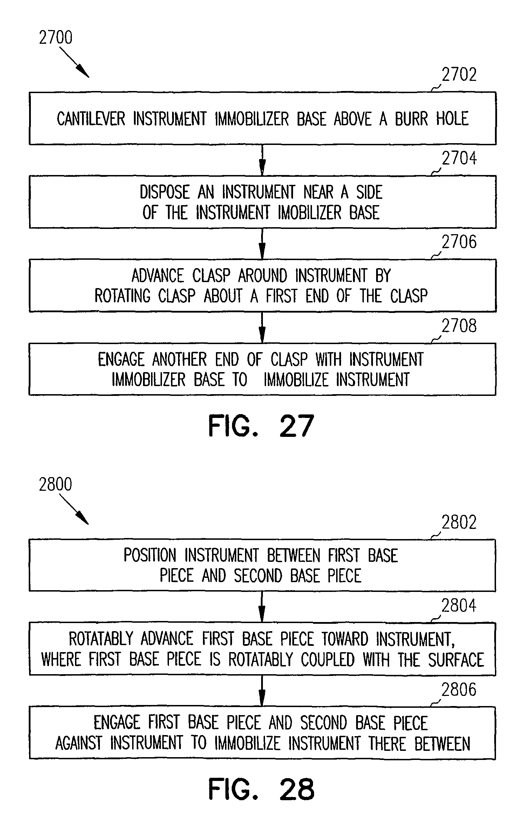

Figure **27** is a block diagram of a method **2700** for immobilizing an instrument. At **2702,** an instrument immobilizer base is cantilevered above a burr hole. At **2704,** the instrument is disposed near a side of the instrument immobilizer base. In one example, the instrument is disposed adjacent a side of the instrument immobilizer base. At **2706** the clasp is advanced around the instrument by rotating the clasp about a first end of the clasp. At **2708,** another end of the clasp is engaged with the instrument immobilizer base to immobilize the instrument.

Several variations are possible. One example includes positioning a portion of the instrument laterally against the instrument immobilizer base, and tucking the instrument under a retaining member overlying the instrument and a groove. Another example includes snap-fitting the clasp to the instrument immobilizer base.

### Method of Use Example 4

Figure **28** is a block diagram of a method **2800** for immobilizing an instrument. At **2802,** an instrument is positioned between a first base piece and a second base piece. At **2804,** the first base piece is rotatably advanced toward the instrument. In one example, the first base piece is rotatably coupled to a surface. At **2806,** the first base piece and the second base piece are engaged against the instrument to immobilize the instrument therebetween.

Several variations are possible. One example includes rotatably advancing the second base piece toward the instrument, where the second base piece is rotatably coupled with the surface. Another example includes snapping the first base piece together with the second base piece. In still another example, a portion of the instrument is positioned within a groove defined by one of the first base piece or the second base piece.

### Method of Use Example 5

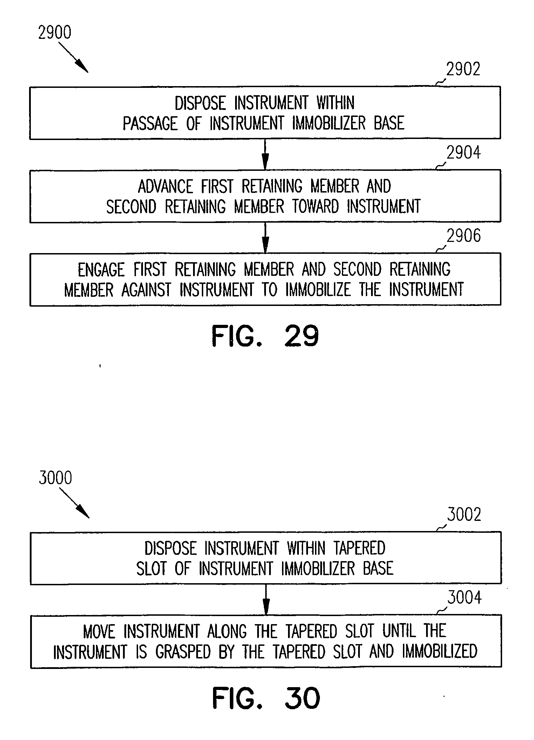

Figure **29** is a block diagram of a method **2900** for immobilizing an instrument. At **2902,** an instrument is disposed within a passage of an instrument immobilizer base. At **2904,** a first retaining member and a second retaining member are advanced toward the instrument. At **2906,** the first retaining member and second retaining member are engaged against the instrument to immobilize the instrument.

Several variations are possible. One example includes coupling an insert with an instrument immobilizer base. In another example, a portion of the instrument is received along edges of the first and second retaining member.

### Method of Use Example 6

Figure **30** is a block diagram of a method **3000** for immobilizing an instrument. At **3002,** an instrument is disposed within a tapered slot of an instrument immobilizer base. At **3004,** the instrument is moved along the tapered slot until the instrument is grasped by the tapered slot and immobilized.

Several variations are possible. One example includes disposing a second instrument within a second tapered slot of the instrument immobilizer base, and moving the second instrument along the second tapered slot until the second instrument is grasped by the second tapered slot and immobilized. In another example, a portion of the instrument is received laterally along the instrument immobilizer base.

### Conclusion

Although the above examples have discussed immobilizing an electrode, these examples are also applicable to immobilizing any other instrument, including, but not limited to instruments for surgical, therapeutic, or diagnostic use, including use in non-medical fields where immobilization of an instrument is advantageous. Some examples of such other medical instruments include, by way of example, but not by way of limitation, a catheter (e.g., for aspiration or for infusion of a drug, cells, or another substance), a probe for measuring pressure, temperature, or some other parameter, a biopsy or other needle. Moreover, certain of the above examples that provide a cover for a burr hole or other entry portal may be useful for serving this function as well, even without securing and immobilizing an instrument. Furthermore, in the above examples, one or more of such components may be coated, impregnated, or otherwise provided with a drug or other therapeutic agent for delivery to the site at which such component(s) are located. Examples of such agents include steroids or other anti-inflammatory agents, or anti-infection agents such as antibiotics or antiviral drugs.

It is to be understood that the above description is intended to be illustrative, and not restrictive. For example, the above-described embodiments may be used in combination with each other. Many other embodiments will be apparent to those of skill in the art upon reviewing the above description. The scope of the invention should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An apparatus (600) comprising:
a base (602), sized and shaped to be disposed above a burr hole, the base (602) includes a slot (606), a first portion of the slot (606) extends between an upper surface and a lower surface of the base (602), and the base (602) includes at least one hole (604a, 604b) sized and shaped to receive a fastener therethrough to secure the base (602); and
a latch (610), rotatably eccentrically coupled to the base (602) above the slot (606), the latch (610) movable between an open position allowing access to the first portion of the slot (606) and a closed position wherein the latch (610) is operable to retain at least one instrument within the first portion of the slot (606) between the base (602) and the latch (610).

2. The apparatus (600) of claim 1, in which the base (602) includes a tab (614) at least partially overlying the slot (606) for securing a portion of the at least one instrument within the slot (606).

3. The apparatus (600) of claims 1 or 2, in which the latch (610) is retained in the closed position by a detent extending from a lower surface of the latch (610) and engaging the base (602).

4. An apparatus (100) comprising:
a base hoop (102), sized and shaped to be disposed about a burr hole, the base hoop (102) includes an upper surface and a lower surface, the base hoop (102) defines an access passage extending between the upper surface and the lower surface of the base hoop (102), the base hoop (102) includes at least one slot opening on a side of the base hoop (102), and at least one hole (104a, 104b) sized and shaped to receive a fastener therethrough to secure the base hoop (102); and
at least one cover piece (106a, 106b), sized and shaped to be inserted through the at least one slot opening to substantially cover the access passage in a closed position, and in the closed position the at least one cover piece (106a, 106b) is operable to retain at least one instrument, the at least one cover piece (106a, 106b) including a catch (109a, 109b) that engages the base hoop (102) when inserted in the slot to hold the at least one cover piece (106a, 106b) in place.

5. The apparatus (100) of claim 4, in which the at least one cover piece (106a, 106b) includes a beveled edge which defines a groove (502) when the at least one cover piece (106a, 106b) is in the closed position, the groove (502) is sized and shaped to retain a portion of the least one instrument and aligns with at least one lateral exit groove.

6. The apparatus (100) of claim 5, in which the base hoop (102) includes the at least one lateral exit groove (114a, 114b) aligned to the groove (502).

7. The apparatus (100) of any one of claims 4 to 6, further comprising:
a second slot opening on an opposed side of the base hoop (102);
a second cover piece (106b) sized and shaped to be inserted through the second slot opening to substantially cover, in the closed position, the access passage together with the at least one cover piece (106a), and in the closed position, the first cover piece (106a) and the second cover piece (106b) are operable to retain the at least one instrument.

8. The apparatus (100) of claim 7, in which the at least one cover piece (106a) and the second cover piece (106b) include respective beveled edges together providing a groove (502) when the at least one cover piece (106a) and the second cover piece (106b) are in the closed position, the groove (502) is sized and shaped to retain a portion of the at least one instrument and aligns with at least one lateral exit groove.

9. The apparatus (100) of any one of claims 4 to 8, in which the at least one cover piece (106a, 106b) includes a catch to secure the at least one cover piece (106a, 106b) in the closed position.

10. An apparatus (1700) comprising:
a base hoop (1702), sized and shaped to be disposed about a burr hole, the base hoop (1700) including an upper surface and a lower surface, the base hoop (1700) defining an access passage between the upper surface and lower surface of the base, the base hoop (1700) including at least one hole (1704a, 1704b) sized and shaped for receiving a fastener therethrough to secure the hoop base (1700); and
an insert (1706), sized and shaped to be positioned within and to engage a surface defining the access passage of the base hoop (1700), the insert (1706) includes an access opening (1708), the insert (1706) also includes a first retaining member (1710a) slidably coupled to the insert (1706) and a second retaining member (1710b) slidably coupled to the insert (1706), the first and second retaining members (1710a), 1710b) sliding toward and away from each other within the access opening (1708), and when in a closed position the first retaining member (1710a) and second retaining member (1710b) engage each other to close the access opening (1708) and are operable to retain at least one instrument therebetween.

11. The apparatus (1700) of claim 10, in which the first retaining member (1710a) includes a beveled edge (1712a) and the second retaining member (1710b) includes a beveled edge (1712b), the beveled edges together defining a groove (1800) when the first retaining member (1710a) and second retaining member (1710b) are in the closed position, the groove (1800) is sized and shaped to retain a portion of the instrument therein and aligns with at least one lateral exit groove.

12. The apparatus (1700) of claim 10 or 11, in which the base hoop (1702) includes the at least one lateral exit groove (1716a, 1716b) aligned to the groove (1800).

## Patentansprüche

1. Vorrichtung (600), die Folgendes umfasst:
eine Basis (602), die dafür bemessen und geformt ist, oberhalb einer Trepanationsöffnung angeordnet zu werden, wobei die Basis (602) einen Schlitz (606) einschließt, wobei sich ein erster Abschnitt des Schlitzes (606) zwischen einer oberen Fläche und einer unteren Fläche der Basis (602) erstreckt, und wobei die Basis (602) wenigstens ein Loch (604a, 604b) einschließt, das dafür bemessen und geformt ist, durch dasselbe ein Befestigungselement aufzunehmen, um die Basis (602) zu befestigen, und
einen Verschluss (610), der oberhalb des Schlitzes (606) drehbar exzentrisch an die Basis (602) gekoppelt ist, wobei der Verschluss (610) bewegt werden kann zwischen einer offenen Stellung, die einen Zugang zu dem ersten Abschnitt des Schlitzes (606) ermöglicht, und einer geschlossenen Stellung, in der der Verschluss (610) funktionsbereit ist, um wenigstens ein Instrument innerhalb des ersten Abschnitts des Schlitzes (606) zwischen der Basis (602) und dem Verschluss (610) festzuhalten.

2. Vorrichtung (600) nach Anspruch 1, wobei die Basis (602) eine Zunge (614) einschließt, die wenigstens teilweise den Schlitz (606) überlagert, um einen Abschnitt des wenigstens einen Instruments innerhalb des Schlitzes (606) zu sichern.

3. Vorrichtung (600) nach Anspruch 1 oder 2, wobei der Verschluss (610) durch eine Arretierung, die sich von einer unteren Fläche des Verschlusses (610) aus erstreckt und die Basis (602) in Eingriff nimmt, in der geschlossenen Stellung festgehalten wird.

4. Vorrichtung (100), die Folgendes umfasst:
einen Basisreifen (102), der dafür bemessen und geformt ist, oberhalb einer Trepanationsöffnung angeordnet zu werden, wobei der Basisreifen (102) eine obere Fläche und eine untere Fläche einschließt, wobei der Basisreifen (102) einen Zugangsdurchgang definiert, der sich zwischen der oberen Fläche und der unteren Fläche des Basisreifens (102) erstreckt, wobei der Basisreifen (102) wenigstens eine Schlitzöffnung auf einer Seite des Basisreifens (102) und wenigstens ein Loch (104a, 104b) einschließt, das dafür bemessen und geformt ist, durch dasselbe ein Befestigungselement aufzunehmen, um den Basisreifen (102) zu befestigen, und
wenigstens ein Abdeckungsstück (106a, 106b), das dafür bemessen und geformt ist, durch die wenigstens eine Schlitzöffnung eingesetzt zu werden, um in einer geschlossenen Stellung den Zugangsdurchgang im Wesentlichen abzudecken, und wobei das wenigstens eine Abdeckungsstück (106a, 106b) in der geschlossenen Stellung funktionsbereit ist, um wenigstens ein Instrument festzuhalten, wobei das wenigstens eine Abdeckungsstück (106a, 106b) eine Klinke (109a, 109b) einschließt, die den Basisreifen (102) in Eingriff nimmt, wenn sie in den Schlitz eingesetzt wird, um das wenigstens eine Abdeckungsstück (106a, 106b) an seinem Platz zu halten.

5. Vorrichtung (100) nach Anspruch 4, wobei das wenigstens eine Abdeckungsstück (106a, 106b) eine abgeschrägte Kante einschließt, die eine Nut (502) definiert, wenn sich das wenigstens eine Abdeckungsstück (106a, 106b) in der geschlossenen Stellung befindet, wobei die Nut (502) dafür bemessen und geformt ist, einen Abschnitt des wenigstens einen Instruments festzuhalten, und mit wenigstens einer seitlichen Ausgangsnut fluchtet.

6. Vorrichtung (100) nach Anspruch 5, wobei der Basisreifen (102) die wenigstens eine seitliche Ausgangsnut (114a, 114b) einschließt, die mit der Nut (502) fluchtet.

7. Vorrichtung (100) nach einem der Ansprüche 4 bis 6, die ferner Folgendes umfasst:
eine zweite Schlitzöffnung auf einer entgegengesetzten Seite des Basisreifens (102),
ein zweites Abdeckungsstück (106b), das dafür bemessen und geformt ist, durch die zweite Schlitzöffnung eingesetzt zu werden, um, in der geschlossenen Stellung, zusammen mit dem wenigstens einen Abdeckungsstück (106a) den Zugangsdurchgang im Wesentlichen abzudecken, wobei das erste Abdeckungsstück (106a) und das zweite Abdeckungsstück (106b), in der geschlossenen Stellung, funktionsbereit sind, um das wenigstens eine Instrument festzuhalten.

8. Vorrichtung (100) nach Anspruch 7, wobei das wenigstens eine Abdeckungsstück (106a) und das zweite Abdeckungsstück (106b) jeweilige abgeschrägte Kanten einschließen, die zusammen eine Nut (502) bereitstellen, wenn sich das wenigstens eine Abdeckungsstück (106a) und das zweite Abdeckungsstück (106b) in der geschlossenen Stellung befinden, wobei die Nut(502) dafür bemessen und geformt ist, einen Abschnitt des wenigstens einen Instruments festzuhalten, und mit wenigstens einer seitlichen Ausgangsnut fluchtet.

9. Vorrichtung (100) nach einem der Ansprüche 4 bis 8, wobei das wenigstens eine Abdeckungsstück (106a, 106b) eine Klinke einschließt, um das wenigstens eine Abdeckungsstück (106a, 106b) in der geschlossenen Stellung zu befestigen.

10. Vorrichtung (1700), die Folgendes umfasst:
einen Basisreifen (1702), der dafür bemessen und geformt ist, oberhalb einer Trepanationsöffnung angeordnet zu werden, wobei der Basisreifen (1702) eine obere Fläche und eine untere Fläche einschließt, wobei der Basisreifen (1702) einen Zugangsdurchgang zwischen der oberen Fläche und der unteren Fläche der Basis definiert, wobei der Basisreifen (1702) wenigstens ein Loch (1704a, 1704b) einschließt, das dafür bemessen und geformt ist, durch dasselbe ein Befestigungselement aufzunehmen, um die Reifenbasis (1702) zu befestigen, und
einen Einsatz (1706), der dafür bemessen und geformt ist, innerhalb einer Fläche angeordnet zu werden und dieselbe in Eingriff zu nehmen, die den Zugangsdurchgang des Basisreifens (1702) definiert, wobei der Einsatz (1706) eine Zugangsöffnung (1708) definiert, wobei der Einsatz (1706) ebenfalls ein erstes Halteelement (1710a), das verschiebbar an den Einsatz (1706) gekoppelt ist, und ein zweites Halteelement (1710b), das verschiebbar an den Einsatz (1706) gekoppelt ist, einschließt, wobei das erste und das zweite Halteelement (1710a, 1710b) innerhalb der Zugangsöffnung (1708) zueinander hin und voneinander weg gleiten und, wenn sie sich in einer geschlossenen Stellung befinden, das erste Halteelement (1710a) und das zweite Halteelement (1710b) einander in Eingriff nehmen, um die Zugangsöffnung (1708) zu schließen, und funktionsbereit sind, um wenigstens ein Instrument dazwischen festzuhalten.

11. Vorrichtung (1700) nach Anspruch 10, wobei das erste Halteelement (1710a) eine abgeschrägte Kante (1712a) einschließt und das zweite Halteelement (1710b) eine abgeschrägte Kante (1712b) einschließt, wobei die abgeschrägten Kanten zusammen eine Nut (1800) definieren, wenn sich das erste Halteelement (1710a) und das zweite Halteelement (1710b) in der geschlossenen Stellung befinden, wobei die Nut (1800) dafür bemessen und geformt ist, einen Abschnitt des Instruments in derselben festzuhalten, und mit wenigstens einer seitlichen Ausgangsnut fluchtet.

12. Vorrichtung (1700) nach Anspruch 10 oder 11, wobei der Basisreifen (1702) die wenigstens eine seitliche Ausgangsnut (1716a, 1716b) einschließt, die mit der Nut (1800) fluchtet.

## Revendications

1. Dispositif (600) comportant :
une base (602), dimensionnée et formée pour être disposée au-dessus d'un trou de trépan, la base (602) inclut une fente (606), une première partie de la fente (606) s'étend entre une surface supérieure et une surface inférieure de la base (602), et la base (602) inclut au moins un trou (604a, 604b) dimensionné et formé pour recevoir une attache à travers celui-ci pour fixer la base (602), et
un verrou (610), couplé à la base (602) de manière rotative et excentrée au-dessus de la fente (606), le verrou (610) étant mobile entre une position ouverte permettant un accès à la première partie de la fente (606) et une position fermée dans laquelle le verrou (610) peut être actionné pour retenir au moins un instrument à l'intérieur de la première partie de la fente (606) entre la base (602) et le verrou (610).

2. Dispositif (600) selon la revendication 1, dans lequel la base (602) inclut une languette (614) recouvrant au moins partiellement la fente (606) pour fixer une partie du au moins un instrument à l'intérieur de la fente (606).

3. Dispositif (600) selon les revendications 1 ou 2, dans lequel le verrou (610) est retenu dans la position fermée par un cran s'étendant depuis une surface inférieure du verrou (610) et en prise avec la base (602).

4. Dispositif (100) comportant :
une bague de base (102), dimensionnée et formée pour être disposée autour d'un trou de trépan, la bague de base (102) inclut une surface supérieure et une surface inférieure, la bague de base (102) définit un passage d'accès s'étendant entre la surface supérieure et la surface inférieure de la bague de base (102), la bague de base (102) inclut au moins une ouverture de fente sur un côté de la bague de base (102), et au moins un trou (104a, 104b) dimensionné et formé pour recevoir une attache à travers celui-ci pour fixer la bague de base (102), et
au moins une pièce de recouvrement (106a, 106b), dimensionnée et formée pour être insérée à travers la au moins une ouverture de fente pour recouvrir sensiblement le passage d'accès dans une position fermée, et dans la position fermée, la au moins une pièce de recouvrement (106a, 106b) peut être actionnée pour retenir au moins un instrument, la au moins une pièce de recouvrement (106a, 106b) incluant une encoche (109a, 109b) qui est en prise avec la bague de base (102) lorsqu'elle est insérée dans la fente pour maintenir la au moins une pièce de recouvrement (106a, 106b) en place.

5. Dispositif (100) selon la revendication 4, dans lequel la au moins une pièce de recouvrement (106a, 106b) inclut un bord biseauté qui définit une gorge (502) lorsque la au moins une pièce de recouvrement (106a, 106b) est dans la position fermée, la gorge (502) est dimensionnée et formée pour retenir une partie du au moins un instrument et est en alignement avec au moins une gorge de sortie latérale.

6. Dispositif (100) selon la revendication 5, dans lequel la bague de base (102) inclut la au moins une gorge de sortie latérale (114a, 114b) alignée sur la gorge (502).

7. Dispositif (100) selon l'une quelconque des revendications 4 à 6, comportant en outre :
une seconde ouverture de fente sur un côté opposé de la bague de base (102),
une seconde pièce de recouvrement (1 06b) dimensionnée et formée pour être insérée à travers la seconde ouverture de fente pour recouvrir sensiblement, dans la position fermée, le passage d'accès en association avec la au moins une pièce de recouvrement (106a), et dans la position fermée, la première pièce de recouvrement (106a) et la seconde pièce de recouvrement (106b) peuvent être actionnées pour retenir le au moins un instrument.

8. Dispositif (100) selon la revendication 7, dans lequel la au moins une pièce de recouvrement (106a) et la seconde pièce de recouvrement (106b) incluent des bords biseautés respectifs créant ensemble une gorge (502) lorsque la au moins une pièce de recouvrement (106a) et la seconde pièce de recouvrement (106b) sont dans la position fermée, la gorge (502) est dimensionnée et formée pour retenir une partie du au moins un instrument et est en alignement avec au moins une gorge de sortie latérale.

9. Dispositif (100) selon l'une quelconque des revendications 4 à 8, dans lequel la au moins une pièce de recouvrement (106a, 106b) inclut une encoche pour fixer la au moins une pièce de recouvrement (106a, 106b) dans la position fermée.

10. Dispositif (1700) comportant :
une bague de base (1700), dimensionnée et formée pour être disposée autour d'un trou de trépan, la bague de base (1700) incluant une surface supérieure et une surface inférieure, la bague de base (1700) définissant un passage d'accès entre la surface supérieure et la surface inférieure de la base, la bague de base (1700) incluant au moins un trou (1704a, 1704b) dimensionné et formé pour recevoir une attache à travers celui-ci pour fixer la bague de base (1700), et
une pièce d'insertion (1706), dimensionnée et formée pour être positionnée à l'intérieur d'une surface définissant le passage d'accès de la bague de base (1700) et pour venir en prise avec celle-ci, la pièce d'insertion (1706) inclut une ouverture d'accès (1708), la pièce d'insertion (1706) inclut également un premier élément de retenue (1710a) couplé de manière glissante à la pièce d'insertion (1706) et un second élément de retenue (1710b) couplé de manière glissante à la pièce d'insertion (1706), les premier et second éléments de retenue (1710a, 1710b) glissant l'un vers l'autre et en s'éloignant l'un de l'autre à l'intérieur de l'ouverture d'accès (1708), et lorsqu'ils sont dans une position fermée, le premier élément de retenue (1710a) et le second élément de retenue (1710b) sont en prise l'un avec l'autre pour fermer l'ouverture d'accès (1708) et peuvent être actionnés pour retenir au moins un instrument entre ceux-ci.

11. Dispositif (1700) selon la revendication 10, dans lequel le premier élément de retenue (1710a) inclut un bord biseauté (1712a) et le second élément de retenue (1710b) inclut un bord biseauté (1712b), les bords biseautés définissant ensemble une gorge (1800) lorsque le premier élément de retenue (1710a) et le second élément de retenue (1710b) sont dans la position fermée, la gorge (1800) est dimensionnée et formée pour retenir une partie de l'instrument à l'intérieur de celle-ci et est en alignement avec au moins une gorge de sortie latérale.

12. Dispositif (1700) selon la revendication 10 ou 11, dans lequel la bague de base (1702) inclut la au moins une gorge latérale de sortie (1716a, 1716b) alignée sur la gorge (1800).
